# EUROPÄISCHE PATENTSCHRIFT

(11) **EP 1 480 033 B1**
(45) Veröffentlichungstag und Bekanntmachung des Hinweises auf die Patenterteilung: **22.07.2009**
(21) Anmeldenummer: 04010763.3
(22) Anmeldetag: 06.05.2004
(51) Int. Cl.: G01N 21/35

(54) **Verfahren zur Bestimmung der Isomerenzusammensetzung bei Isocyanat-Herstellprozessen**
Method for determining the content of isomeres during production of isocyanate
Méthode pour déterminer la constitution des isomères pendant la production d'isocyanate

(30) Priorität: 19.05.2003 DE 10322439
(43) Veröffentlichungstag der Anmeldung: 24.11.2004
(73) Patentinhaber: Bayer MaterialScience AG, 51368 Leverkusen (DE)
(72) Erfinder: Mahrenholtz, Jochen, 47802 Krefeld (DE); Wimschneider, Andrea, Dr., 40597 Düsseldorf (DE); Pirkl, Hans-Georg, Dr., 51377 Leverkusen (DE); Müller, Heinz-Herbert, Dr., 47809 Krefeld (DE); Dresely, Stefan, Dr., 47802 Krefeld (DE); Bolton, Jeffrey, Dr., Baytown, TX 77520 (US); Schiffhauer, Martin, 40699 Erkrath (DE); Wolf, Udo, Dr., 47906 Kempen (DE); Schweer, Johannes, Dr., 51061 Köln (DE); Gerlach, Martin, Dr., 41539 Dormagen (DE)

(56) Entgegenhaltungen:
- EP-A- 0 644 421
- DD-A- 273 175
- DE-A- 10 005 130
- US-A- 6 072 576
- US-A- 6 162 644
- US-B1- 6 300 633

## Beschreibung

Die Erfindung betrifft das Gebiet der Bestimmung der Isomerenzusammensetzung bei Isocyanat-Herstellprozessen sowie die Regelung einer Isomeren-Anlage für die Mischung oder die Trennung von Isomeren.

Aus dem Stand der Technik ist es bekannt, ein Isomerengemisch von Isocyanaten einer bestimmten Isomerenzusammensetzung mittels einer Isomerenanlage herzustellen.

Beispielsweise kann eine Isomerentrennung destillativ oder mittels Kristallisation erfolgen. Oder die Herrstellung eines bestimmten Isomerengemisches erfolgt durch Mischen von geeigneten Isomerenausgangsmischungen. Die Qualität kann beispielsweise in einer destillativen Isomerentrennung grob über die Prozessparameter Druck und Temperatur sowie die Destillat/Sumpfproduktverhältnisse und Rücklaufverhältnisse geregelt werden. Hierbei ist von Nachteil, dass beispielsweise bei hohen Produktreinheiten Druck und Temperatur keine praktisch nutzbare Information über die Konzentration der Isomeren liefern; d. h. die Sensitiviät der Konzentrationsbestimmung liegt im Bereich des Messrauschens aufgrund der eng beieinander liegenden Siedepunkte. Weiterhin ist bei einer Isocyanat-Isomerenmischung bisher keine physikalische Methode zur Bestimmung von Isomerenmischungen bekannt.

Bisher wurde diese Qualitätsüberwachung deshalb per Probenahme und z. B. anschließender manueller chromatographischer Analytik, bevorzugt Gaschromatographie (GC) durchgeführt. Hierbei sind Arbeitsschutz- und Umweltschutzbedingungen zu beachten, um Gefährdungen aufgrund des Umgangs mit den chemischen Stoffen zu vermeiden; ferner ist die Probenanzahl wegen des personellen Aufwandes begrenzt und die Information über die Zusammensetzung der Probe steht erst mit einer erheblichen Verzögerung zur Verfügung. Zur Steuerung der Produktqualität von Kristallisatoren oder Destillationskolonnen hat dieses manuelle Verfahren daher erhebliche Nachteile, insbesondere da sie keine laufende Trendaussage über die Konzentrationsänderungen an vielen Apparaten einer komplexen Anlage zulässt.

Bei solchen manuellen Steuerungen kann es daher während relativ langer Zeiträume dazu kommen, dass das hergestellte Isomerengemisch eine relativ große Abweichung von der Soll-Zusammensetzung aufweist. Dies kann die Reduzierung der Produktqualität oder die Herstellung von Ausschuss bedeuten.

Als online Verfahren kommen Prozess-Chromatographie oder automatisierte Titration in Frage. Diesen Verfahren gemeinsam ist, dass das Ergebnis nur erheblich zeitverzögert nach längeren Messzeiten zur Verfügung steht. Daneben sind diese Verfahren durch aufwändige Probenzuführung, Störanfälligkeit und größeren Verbrauch an Hilfsstoffen und Verbrauchsmaterialien gekennzeichnet.

Die Überwachung und Regelung der Isomerenzusammensetzung ist insbesondere für die Herstellung von Isocyanaten von Bedeutung. Verschiedene Isocyanate A, B, C, D usw. bestehen dabei aus einer Mischung von zwei oder mehreren Isomeren 1, 2, 3, ..., n.

Diese Isocyanate können z. B. Naphthylen-Diisocyanate (Bis-[isocyanat]-naphthalin), Xylylen-Diisocyanate (Bis-[isocyanatmethyl]-benzol), Methylen-Diphenyl-Diisocyanate (MDI) oder Toluylen-Diisocyanate (TDI) sein, sowie weitere aromatische, alicyclische oder aliphatische Isocyanate und deren Mischungen.

Im allgemeinen bestehen Isocyanat-Zwischen- oder -Verkaufsprodukte aus den verschiedenen Isomeren in unterschiedlichen Verhältnissen.

Technisch werden solche Isocyanat-Zwischen- oder -Verkaufsprodukte aus einem Isocyanat-Ausgangsgemisch (Rohgemisch) mehrerer Isomere 1,2,3, ..., n hergestellt.

Beispielsweise kann das Isocyanat A Toluylendiisocyanat (TDI) sein, ein Isomerengemisch aus den Isomeren 2,4-TDI (2,4-Bis-[isocyanat]-benzol), 2,6 -TDI (2,6-Bis-[isocyanat]-benzol), 2,3-TDI und 3,4-TDI. Das Ausgangsgemisch kann zur Erzielung spezieller hochwertiger Produkteigenschaften in seine Isomeren zerlegt werden. So sind auf dem Markt ein Verkaufsprodukt I mit ca. 100 % 2,4-TDI, sowie ein Verkaufsprodukt II mit ca. 65 % 2,4-TDI und ca. 35 % 2,6-TDI erhältlich.

Beispielsweise kann das Isocyanat B Methylen-Diphenyl-Diisocyanat (MDI) sein, ein Isomerengemisch aus den Isomeren 2,2'-MDI (Bis-[2-isocyanat-phenyl]-methan), 2,4'-MDI (2-Isocyanat-phenyl)-(4-isocyanat-phenyl)-methan 4,4'-MDI (Bis-[4-isocyanat-phenyl]-methan) sowie weiterer höherkerniger Isomere.

Das Ausgangsgemisch kann zur Erzielung spezieller hochwertiger Produkteigenschaften in seine Isomeren zerlegt werden. So sind auf dem Markt ein Verkaufsprodukt I mit ca. 100 % 4,4'-MDI, sowie ein Verkaufsprodukt II mit ca. 50 % 2,4'-MDI und ca. 50 % 4,4'-MDI erhältlich.

Eine möglichst genaue Überwachung der Isomerenzusammensetzung ist für die Einhaltung einer vorgegebenen Produktspezifikation zwingend erforderlich. Diese Überwachung muss die Zusammensetzung möglichst schnell liefern, damit die Nachregelung der Isomerenanlage effizient erfolgen kann. Da es sich bei der Isocyanat-Isomerenherstellung produktionsbedingt um Koppelprodukte handeln kann, ist eine schnelle und möglichst präzise Überwachung besonders wichtig.

Die bisher praktizierten Verfahren können diese Anforderungen nur mit erheblichen Einschränkungen erfüllen. Beispielsweise müssen für die offline gaschromatographische Untersuchung Proben gezogen und ins Labor transportiert werden, dort müssen die Probe aufbereitet und anschließend gaschromatographisch analysiert werden.

Als Alternative zur Gaschromatographie und Titration sind zur quantitativen Analyse der Zusammensetzung von Stoffgemischen aus dem Stand der Technik spektroskopische Verfahren bekannt, beispielsweise Nah-Infrarot(NIR)-Spektroskopie, Mittelinfrarot-Spektroskopie und Raman-Spektroskopie.

Das analytische Verfahren Nahinfrarot-(NIR-)Spektroskopie ist eine weit verbreitete Technik, die sowohl im Labor als auch im Online-Betrieb eingesetzt wird. Die Kombination von NIR-Spektroskopie mit chemometrischen Auswerteverfahren für spezielle Messaufgaben ist ebenfalls an sich aus dem Stand der Technik bekannt, z. B. DE 02139269, WO 97/41420, WO 98/29787, WO 99/31485, JP 11350368, WO 20002/0834, JP 2000146835, JP 2000298512, WO 2002/04394, WO 2002/12969, WO 95/31709, U.S. Patent 5,707,870, U.S. Patent 5,712,481, und WO 2000/68664.

Spektroskopische Analysetechniken, zur Bestimmung von chemischen Eigenschaften von Polymeren oder von physikalischen Eigenschaften von Polyurethan-Schäumen sowohl im Labor als auch im Online-Betrieb, sind bekannt aus "A review of process near infrared spectroscopy: 1980-1994" (J. Workman, J. Near Infrared Spectroscopy 1, 221-245 (1993). Die Vorteile der Kombination aus Lichtwellenleitern und NIR-Spektrometer gegenüber dem Einsatz der Mittel-Infrarot-Spektroskopie sind bekannt aus Khettry "In-Line Monitoring of Polymeric Processes" Antec '92, 2 674-2 676.

Für den Einsatz der NIR-Spektrokopie im Bereich von quantitativen Bestimmungen wird das analytische Verfahren häufig in Kombination mit chemometrischen Auswertemethoden verwendet. Gebräuchlich ist dabei z. B. das Partial Least Square-Verfahren (PLS), wie z. B. aus Raphael Vieira "In-line and In Situ Monitoring of Semi-Batch Emulsion Copolymerizations Using Near-Infrared Spectroscopy" J Applied Polymer Science, Vol. 84, 2 670-2 682 (2002) ersichtlich, oder aus T. Rohe "Near Infrared (NIR) spectroscopy for inline monitoring of polymer extrusion processes" Talanta 50 (1999) 283-290 bzw. C. Miller "Chemometrics for on-line spectroscopy applications - theory and practice", J Chemometrics 2000; 14: 513-528 bzw. "Multivariate Analysis of Near-Infrared Spectra Using G-Programming Language" J. Chem. Inf.Comput. Sci. 2000, 40, 1 093-1 100.

Die Verwendung von NIR-Techniken für spezielle Messaufgaben ist ferner bekannt aus: WO 00/02035 (Bestimmung von organischer Säure in organischem Polymer), US 005717209 (Spektralanalyse von Kohlenwasserstoffen), US 006228650; WO 99/31485 (Kontrolle der Trennung von chemischen Komponenten in einem Alkylierprozess mit Säurekatalysator), US 6339222; WO 00/68664 (Bestimmung von Ionischen Spezies in Pulp Liquor), DE 10005130A1 (Kontrolle von Polymerprozessen, Bestimmung von NCO in PU).

Einen Überblick über die Verwendung von multivariaten chemometrischen Kalibrationsmodellen in der analytischen Chemie gibt ferner "Multivariate Kalibration", Jörg-Peter Conzen, 2001, ISBN 3-929431-13-0.

Die US 6162644 beschreibt die Isomeren-Trennung u.a. durch Konzentrationsmessung von Xylenen durch Vergleich von NIR-Spektren.

Die DE 10005130 A1 offenbart ein Verfahren zur Kontrolle von Polymerisationsreaktionen von Isocyanaten durch Messung von Absorptionsbanden im nahen Infrarotbereich.

Im Stand der Technik werden solche spektroskopische Verfahren für Isocyanat-Isomergemische jedoch nicht eingesetzt.

Der Erfindung liegt daher die Aufgabe zugrunde, ein verbessertes Verfahren zur Bestimmung der Isomerenzusammensetzung in einem Isocyanat-Isomerengemisch sowie ein verbessertes Verfahren zur Regelung einer Isomerenanlage.

Die der Erfindung zugrunde liegenden Aufgaben werden jeweils mit den Merkmalen der unabhängigen Patentansprüche gelöst. Bevorzugte Ausführungsformen der Erfindung sind in den abhängigen Patentansprüchen angegeben.

Ausgangspunkt der vorliegenden Erfindung ist die Entdeckung, dass sich die Absorptionsspektren von Isocyanat-Isomerengemischen überraschenderweise selbst bei kleinen Konzentrationsunterschieden und bei kleinen Gehalten einzelner Isomere hinreichend voneinander unterscheiden, um mit Hilfe eines chemometrischen Kalibrationsmodells die Isomerenkonzentrationen in einem Isomerengemisch auf der Grundlage der Messung des Spektrums des Isomerengemisches bestimmen zu können.

Erfindungsgemäß erfolgt die Bestimmung von Methylen-Diphenyl-Diisocyanat (MDI)-Isomeren Konzentrationen gemäß Anspruchs 1. Die Bestimmung der Zusammensetzung in einem Isocyanat-Isomerengemisch erfolgt so, dass beispielsweise online ein Spektrum des Isomerengemisches mit einem optischen Sensor mittels Nah-Infrarot(NIR)-Spektroskopie, Mittelinfrarot-Spektroskopie oder Raman-Spektroskopie aufgenommen wird. Das gemessene Spektrum wird dann in ein chemometrisches Kalibrationsmodell eingegeben, was zuvor für die Mischung dieser Isomeren erstellt worden ist. Durch die Auswertung des Spektrums in dem chemometrischen Kalibrationsmodell erhält man die Isomerenkonzentrationen in dem Isomerengemisch. Durch Vergleich der so ermittelten Ist-Konzentrationen der Isomere mit spezifizierten Soll-Konzentrationen kann die Isomerentrennanlage entsprechend nachgeregelt werden. Das chemometrische Kalibrationsmodell kann z. B. ein multivariates Modell sein, z. B. ein Partial Least Square-Algorithmus.

Von besonderem Vorteil ist hierbei, dass die Konzentrationsmessungen ständig, d. h. beispielsweise in kleinsten Zeitabständen, erfolgen können und somit eine Regelung der Isomerenanlage innerhalb enger Sollwertbereichen möglich ist. Insbesondere ist so die Produktion von Ausschuss oder geringer Produktqualität weitgehend vermeidbar.

Ein weiterer Vorteil ist, dass die manuelle Probenentnahme und GC-Analytik entfallen kann. Dies hat insbesondere Vorteile hinsichtlich Arbeits- und Umweltschutz.

Von besonderem Vorteil ist ferner, dass die Aufnahme der Spektren online/inline an einer oder mehrerer verschiedener Stellen einer komplexen Anlage erfolgen kann, und zwar ohne Probenentnahme. Eine solche komplexe Anlage besteht beispielsweise aus mehreren miteinander verschalteten Apparaten, z. B. Kolonnen oder Kristallisatoren. Dies ermöglicht eine zeitnahe und häufige Information über die Isomerenkonzentrationen. Diese Information kann für eine manuelle Regelung eines oder mehrerer der Parameter der Isomerenanlage verwendet werden oder auch für eine kontinuierliche, automatische Regelung der Produktionsanlage.

Beispielsweise können mehrere optische Sensoren zur Aufnahme von Spektren an verschiedenen Stellen einer Isomerenanlage zur Aufnahme der Spektren verschiedener Isomerengemische angeordnet sein. Diese optischen Sensoren können z. B. über Glasfaser mit einem einzigen Spektrometer verbunden sein, welches im Multiplexbetrieb arbeitet. Dadurch wird der investive Aufwand minimiert. Ferner können aufgrund dieser Online-Analytik Probenentnahme-Leitungen bis zu einem Analysegerät mit der Gefahr von Verstopfungen durch Auskristallisieren und dergleichen vermieden werden. Dies ist ein besonderer Vorteil der Erfindung, da solche Analysenleitungen störanfällig sind und ein zusätzliches Handling der anfallenden Produkt-Analysenstoffströme erfordern.

Ein weiterer Vorteil ist die Möglichkeit der automatischen Prozessführung auf der Grundlage der Konzentrationsinformationen mit der dadurch möglichen Einhaltung einer nahezu konstanten Produktqualität mit geringem Energieaufwand bei hoher Ausbeute sowie das konsequente Ausschöpfen der Anlagenkapazität.

Von weiterem besonderen Vorteil ist die universelle Einsetzbarkeit der Erfindung für verschiedenste Isomerenzusammensetzungen bei Isocyanat-Herstellprozessen. Insbesondere sind mit Hilfe der vorliegenden Erfindung hohe Messgenauigkeiten für die Bestimmung der Isomerenzusammensetzung erreichbar, wenn einer oder mehrere der Isomere in ganz geringen Konzentrationen vorliegen, als auch wenn die Mischungskonzentrationen in etwa gleichgewichtig sind. Beispielsweise können mit Hilfe der vorliegenden Erfindung Konzentrationsmessungen eines Isomers in einem Isomerengemisch durchgeführt werden, wenn die Konzentration des Isomers zwischen 0,01 % und 99,99 % liegt. Einige bevorzugte Anwendungen für die Bestimmung der Isomerenzusammensetzung bei Isocyanat-Herstellprozessen sind im Folgenden angegeben:
a) 0-3% 2,4'-MDI, , 0-3% 2,2'-MDI, Rest 4,4'-MDI
b) 40% - 70% 2,4'-MDI, 0% - 3% 2,2'-MDI, Rest 4,4'-MDI

Weitere Anwendungen für die Bestimmung der Isomerenzusammensetzung bei Isocyanat-Herstellprozessen sind: 0-40% 2,6-TDI, Rest 2,4-TDI.

Im Weiteren werden Ausführungsformen mit Bezugnahme auf die Zeichnungen näher erläutert. Es zeigen:
- Figur 1: ein Flussdiagramm einer Ausführungsform eines erfindungsgemäßen Verfahrens zur Bestimmung der Isomerenzusammensetzung in verschiedenen Isomerengemischen,
- Figur 2: ein Blockdiagramm einer Isomerentrennanlage,
- Figur 3: ein Blockdiagramm einer Isomerenmischanlage,
- Figur 4: verschiedene Spektren von MDI-Isomeren,
- Figur 5: die Spektren von zwei unterschiedlichen MDI-Isomerengemischen,
- Figur 6: das Differenzspektrum der Spektren der Figur 5,
- Figur 7: verschiedene Spektren von TDI-Isomeren,
- Figur 8: eine Kalibrationskurve für die Bestimmung von 2,4-TDI in 2,6-TDI.

In dem Schritt 100 erfolgt die Herstellung eines Isomerengemisches. Bei dem Gemisch handelt es sich beispielsweise um rohes MDI-Gemisch, welches aus drei MDI-Isomeren besteht oder um rohes TDI-Gemisch, welches aus bis zu vier TDI-Isomeren besteht..

In dem Schritt 102 werden aus dem Roh-Isomerengemisch, welches in dem Schritt 100 hergestellt worden ist, ein oder mehrere Gemische Gᵢ, die jeweils eine bestimmte Soll-Isomerenzusammensetzung aufweisen, durch Isomerentrennung hergestellt. Dies erfolgt in einer Isomerentrennanlage, beispielsweise destillativ oder mittels Kristallisation. Ebenfalls kann es sich statt um eine Isomerentrennanlage um eine Isomerenmischanlage handeln.

In dem Schritt 104 wird der Index i initialisiert. In dem Schritt 106 wird online das Spektrum des Gemisches Gᵢ am Ausgang der Isomerenanlage gemessen. Dies erfolgt beispielsweise durch eine Online-NIR-Spektralmessung. Die Messung erfolgt beispielsweise durch einen optischen Sensor, der mittels eines Glasfaserkabels mit einem NIR-Spektrometer verbunden ist.

In dem Schritt 108 erfolgt eine Spektralanalyse des gemessenen NIR-Spektrums mit Hilfe eines chemometrischen Kalibrationsmodells. Daraus resultiert in dem Schritt 110 die Ist-Isomerenzusammensetzung des Gemisches Gᵢ. In dem Schritt 112 wird aus den Soll- und den Ist-Werten der Isomerenzusammensetzung des Gemisches Gᵢ die Differenz gebildet. Auf der Basis dieses Differenzwertes erfolgt in dem Schritt 114 eine Nachregelung der Isomerenanlage. In dem Schritt 116 wird der Index i inkrementiert und das nächste Spektrum wird in dem Schritt 106 gemessen. Dieser Ablauf wiederholt sich solange, bis die Ist-Isomerenzusammensetzung für alle Gemische Gᵢ einmal bestimmt worden ist. Danach wird der Index i zurückgesetzt, sodass kontinuierlich innerhalb relativ kurzer Zeitabstände, von z. B. wenigen Minuten die Ist-Isomerenzusammensetzungen sämtlicher Gemische Gᵢ bestimmt werden und eine entsprechende Nachregelung zeitnah erfolgen kann.

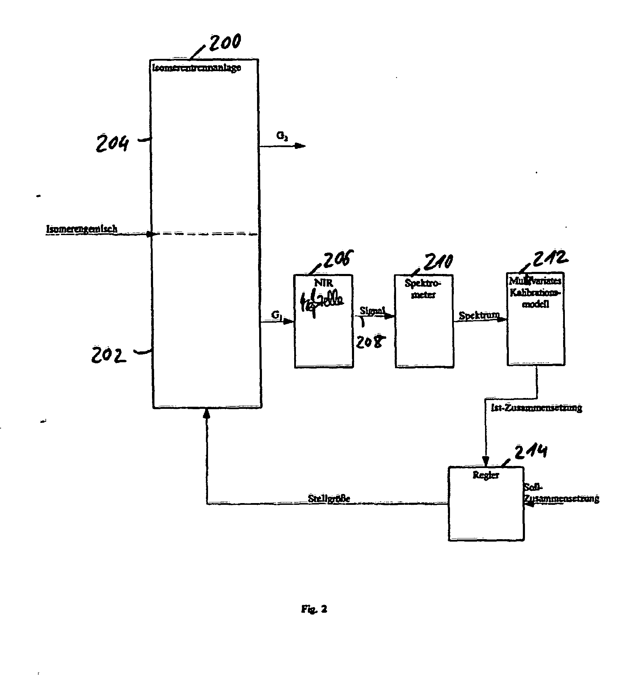

Figur 2 zeigt eine Isomerentrennanlage 200 zur Herstellung von Isomeren-Gemischen G₁ und G₂ mit jeweils definierten Isomerenkonzentrationen aus einem Roh-Isomerengemisch. Beispielsweise erhält man in einem Sumpfbereich 202 der destillativen Isomerentrennanlage 200 das Gemisch G, und in einem Kopfbereich 204 der Isomerentrennanlage 200 das Gemisch G₂.

Zumindest am Ausgang des Sumpfbereichs 202 der Isomerentrennanlage 200 ist ein optischer Messzelle 206 angeordnet. Die Messzelle 206 beinhaltet einen NIR-Sensor und kann beispielsweise mit dem in WO 00/58711 beanspruchten "Druckfesten Prozessfenster" ausgebildet sein. Der Messzelle 206 ist vorzugsweise über einen Lichtwellenleiter 208 mit einem Spektrometer 210 verbunden. Das Spektrometer 210 liefert ein Spektrum, welches in ein chemometrisches Kalibrationsmodell 212 eingegeben wird. Das chemometrische Kalibrationsmodell 212 kann durch eine separate Auswerteeinheit, beispielsweise einen handelsüblichen PC realisiert werden. Alternativ kann auf das Spektrometer 210 selbst eine solche Auswerteeinheit für das Spektrum beinhalten.

Als Ergebnis der Analyse des gemessenen Spektrums gibt das chemometrische Kalibrationsmodell 212 die Ist-Zusammensetzung des Isomerengemisches G₁ aus. Diese Ist-Zusammensetzung wird in einen Regler 214 eingegeben sowie auch die Soll-Zusammensetzung des Isomerengemischs G₁. Aus einer Abweichung zwischen der Ist-Zusammensetzung und der Soll-Zusammensetzung ermittelt der Regler 214 eine Stellgröße für die Nachregelung der Isomerenanlage 200.

Bei der Ausführungsform der Figur 2 ist eine Messung des Spektrums des Gemischs G₂ nicht unbedingt erforderlich, da sich die Isomerenkonzentrationen in dem Gemisch G₂ aus den bekannten Konzentrationen in dem Roh-Isomerengemisch und der Ist-Zusammensetzung des Gemischs G₁ ergibt.

Andererseits können an verschiedenen Stellen der Isomerentrennanlage 200, insbesondere an verschiedenen Kolonnen, weitere Sensoren 206 angeordnet sein, die wiederum über Lichtwellenleiter mit dem Spektrometer 210 verbunden sind. Das Spektrometer 210 wird in diesem Fall im Multiplex betrieben ebenso wie das chemometrische Kalibrationsmodell 212 und der Regler 214. Die Messung der Spektren kann also an verschiedenen Stellen der Isomerentrennanlage 200 erfolgen, vorzugsweise in den Einläufen, innerhalb des Trennapparats und / oder in einer getrennten Fraktion.

Bei dem Isomerengemisch handelt es sich beispielsweise um rohes Monomer-MDI, welches aus den drei Isomeren 2,4'-MDI, 2,2'-MDI und 4,4'-MDI besteht.

Der Regler 214 kann durch ein Prozessleitsystem der Isomerentrennanlage 200 realisiert werden. Alternativ ist es auch möglich, die Messergebnisse beispielsweise auf eine Anzeigeinheit einer Bedienkonsole der Isomerentrennanlage 200 anzuzeigen, so dass diese manuell nachgeregelt werden kann.

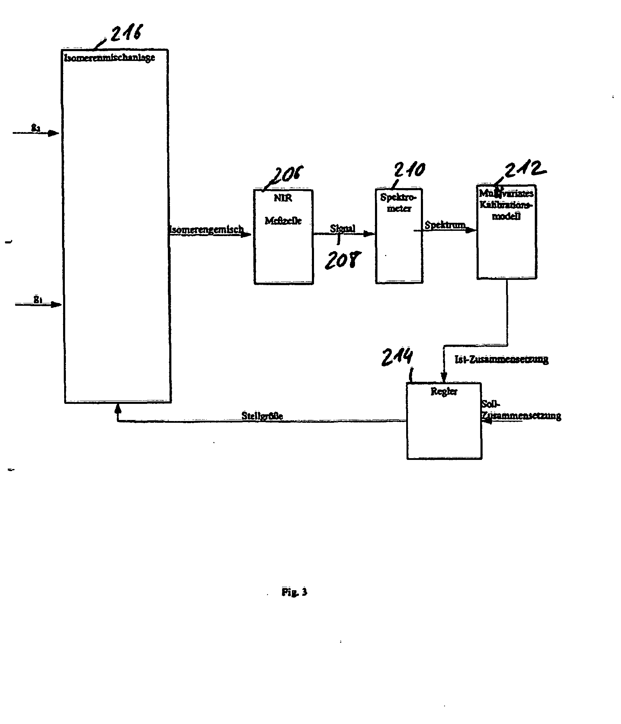

Die Figur 3 zeigt eine Isomerenmischanlage 216. Elemente der Isomerenmischanlage 216, die den Elementen der Isomerentrennanlage 200 entsprechen, sind in der Figur 3 mit denselben Bezugszeichen gekennzeichnet. Die Isomerenmischanlage 216 dient zur Herstellung eines Isomerengemisches mit einer bestimmten Soll-Zusammensetzung aus verschiedenen Isomeren. Für das in der Isomerenmischanlage 216 hergestellte Isomerengemisch wird mittels der Messzelle 206, die über einen Lichtwellenleiter 208 mit einem Spektrometer 210 verbunden ist, ein Spektrum ermittelt, welches in ein chemometrisches, multivariates Kalibrationsmodell 212 eingegeben wird. Als Ergebnis der Analyse des gemessenen Spektrums gibt das chemometrische Kalibrationsmodell 212 die Ist-Zusammensetzung des Isomerengemisches aus. Diese Ist-Zusammensetzung wird in einen Regler 214 eingegeben, sowie auch die Soll-Zusammensetzung des Isomerengemisches. Aus einer Abweichung zwischen der Ist-Zusammensetzung und der Soll-Zusammensetzung ermittelt der Regler 214 eine Stellgröße für die Nachregelung der Isomerenmischanlage 216.

Für die Herstellung des Isomerengemischs werden der Isomerenmischanlage zwei oder mehr Reinisomere oder Isomerengemische g₁, g₂, ... zugeführt. Hierbei kann es sich beispielsweise um TDI-Reinisomere und / oder um TDI-Isomerengemische handeln. Zur Überwachung der Zusammensetzungen der Isomeren- bzw. Isomerengemische g₁, g₂, ... können an den entsprechenden Einläufen der Isomerenmischanlage jeweils Messzellen 206 angeordnet sein, die ebenfalls mit dem Spektrometer 210 verbunden sind, um die Ist-Zusammensetzungen der Ausgangsstoffe g₁, g₂, ... zu überwachen.

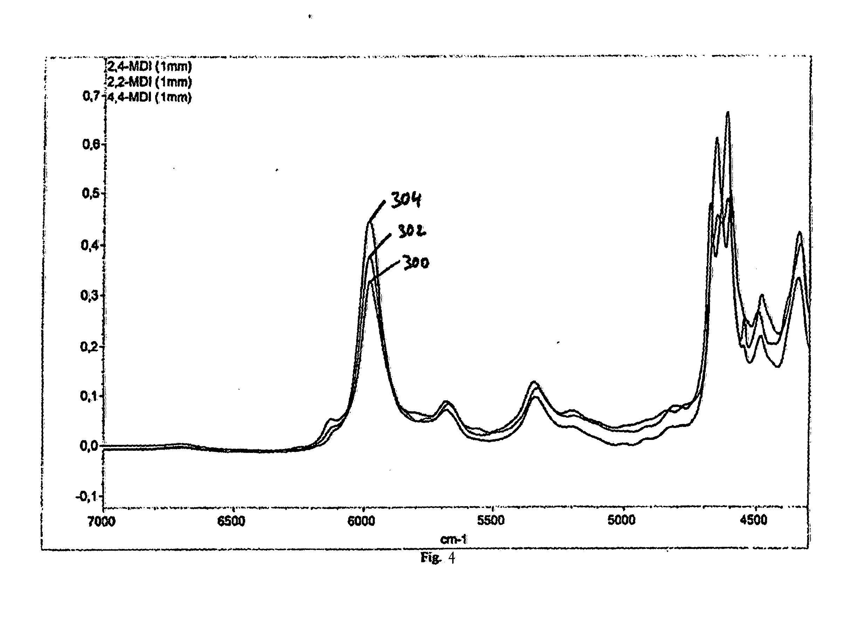

Die Figur 4 zeigt Beispiele für die entsprechenden Spektren, d. h. das Spektrum 300 für reines 4,4'-MDI, das Spektrum 302 für 2,4'-MDI und das Spektrum 304 für 2,2'-MDI. Wie aus der Figur 4 ersichtlich, sind diese Spektren 300, 302, 304 ähnlich.

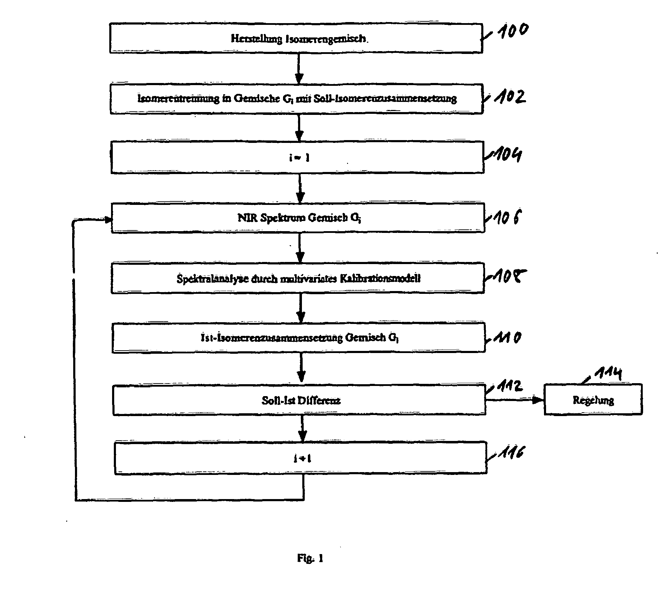

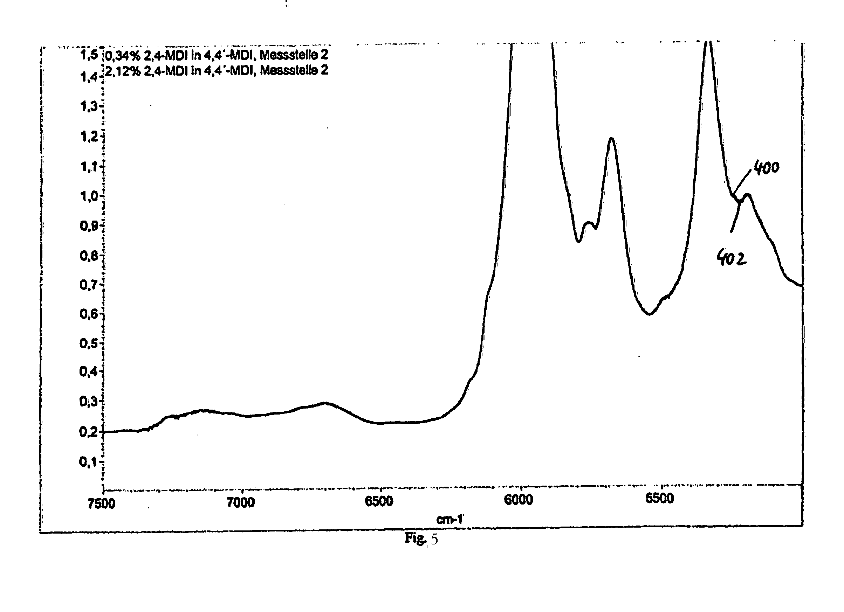

Die Figur 5 zeigt das Spektrum 400 eines Isomerengemisches aus 0,34 % 2,4'-MDI in 4,4'-MDI. Dieses Gemisch erhält man beispielsweise als Gemisch G₁ am Sumpfbereich 202 der Isomerentrennanlage (vergleiche Figur 1). Ferner zeigt die Figur 5 das Spektrum 402, des Isomerengemischs von 2,12 % 2,4'-MDI in 4,4'-MDI. Wie aus der Figur 5 ersichtlich, sind die Spektren dieser Isomerengemische nahezu deckungsgleich.

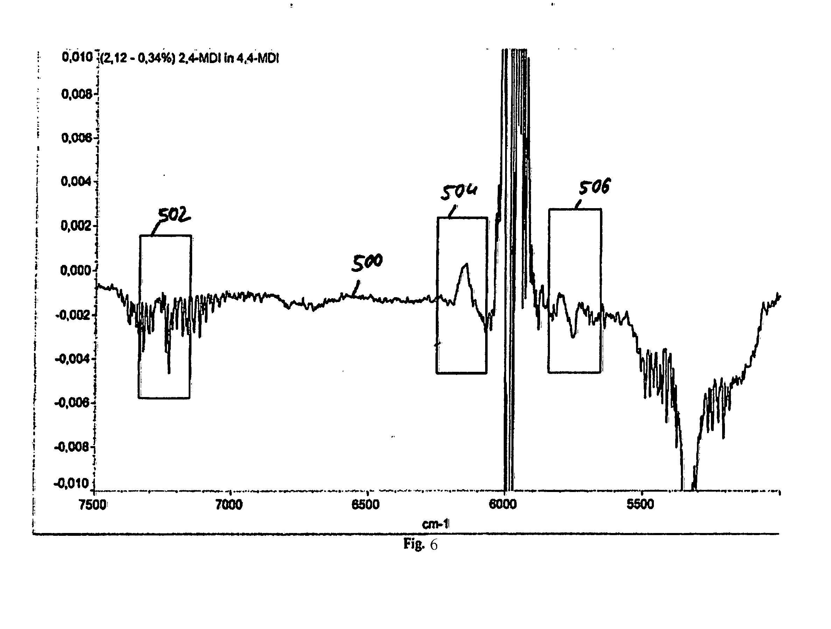

Die Figur 6 zeigt das Differenzspektrum aus den Spektren 402 und 400 der Figur 5. Vorzugsweise werden nur die Frequenzbereiche 502 und/oder 504 und/oder 506 für die Auswertung in dem multivariaten Kalibrationsmodell 212 (vergleiche Figur 2) verwendet. Bevorzugte Frequenzbereiche für die Auswertung des Spektrums sind insbesondere die Frequenzbereiche von 5000-7000 cm-1, bevorzugt 6250-6080 cm-1 oder 5840-5650 cm-1. Damit lassen sich Isomerenkonzentrationen in einem Isomerengemisch z. B. im Bereich von 0,01 % bis 99,99 % bestimmen, mit einer Genauigkeit von kleiner 0,1% absolut. Ein Reglereingriff kann also bereits bei einer Sollwert-Abweichung von nur 0,1 % erfolgen, sodass eine praktisch gleich bleibende Produktqualität zu jedem Zeitpunkt gewährleistet ist, insbesondere bei einer vollautomatischen Regelung.

Die Absorptionsspektren werden entweder als Originalspektren, oder als erste, zweite oder höhere Ableitungsspektren weiterverarbeitet. Bevorzugt werden die ersten Ableitungsspektren weiterverarbeitet.

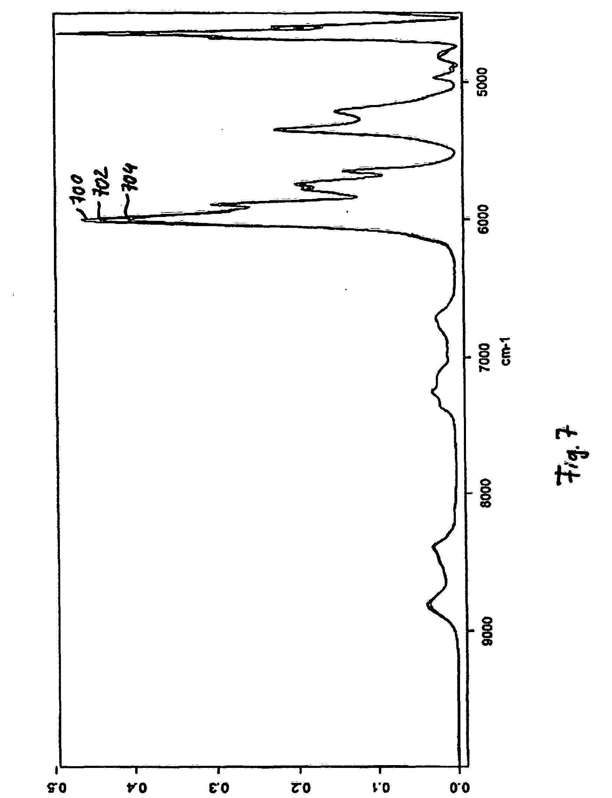

Die Figur 7 zeigt die entsprechenden Spektren für verschiedene Gemische von 2,4-TDI und 2,6-TDI. Für ein Isomerengemisch aus 67 % 2,4 TDI mit Rest 2,6-TDI erhält man des Spektrum 700; für 81 % 2,4 TDI mit Rest 2,6 TDI erhält man das Spektrum 702; für eine Konzentration von ≥ 99,5 2,4 TDI mit einem Rest von 2,6-TDI erhält man das Spektrum 704. Wie aus der Figur 7 ersichtlich, sind diese Spektren 700, 702. und 704 sehr ähnlich. Dennoch wird ermöglicht insbesondere auch kleine Konzentrationsunterschiede genau zu bestimmen.

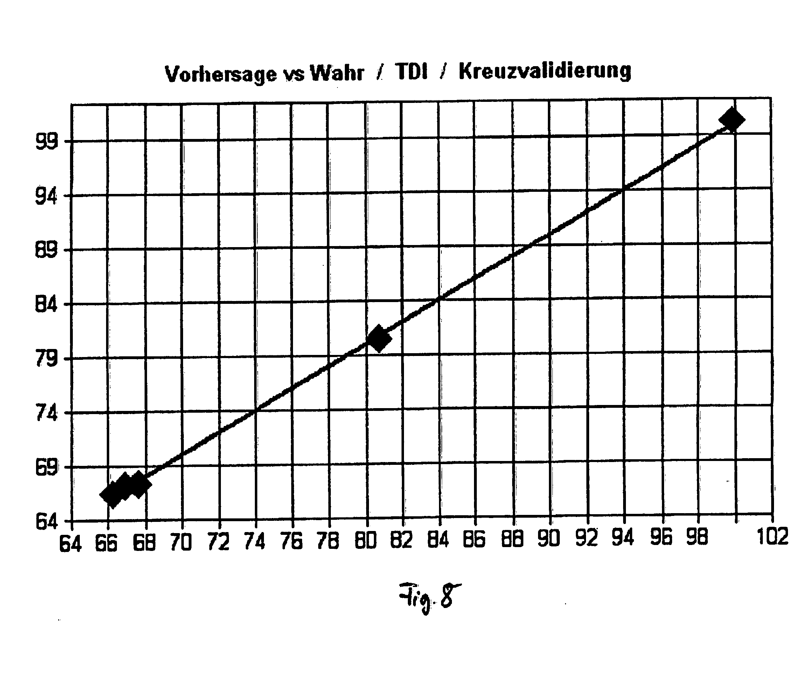

Figur 8 zeigt die Kalibrationskurve für die Bestimmung von 2,4-TDI in 2,6-TDI. Hieraus ist ersichtlich, dass die quantitative Analyse mit einer großen Genauigkeit möglich ist, trotz der Ähnlichkeit der Spektren. Bevorzugte Frequenzbereiche für die Auswertung des Spektrums sind insbesondere die Frequenzbereiche von 4500-9000 cm-1, bevorzugt 5610-6220 cm-1 sowie von 5240-5480 cm-1.

### Bezugszeichenliste

- Isomerentrennanlage: 200
- Sumpfbereich: 202
- Kopfbereich: 204
- Messzelle: 206
- Lichtwellenleiter: 208
- Spektren: 210
- chemometrisches Kalibrationsmodell: 212
- Regler: 214
- Isomerenmischanlage: 216
- 4,4'-MDI-Spektrum: 300
- 2,4'-MDI-Spektrum: 302
- 2,2'-MDI-Spektrum: 304
- Spektrum: 400
- Spektrum: 402
- Spektrum: 500
- Frequenzbereich: 502
- Frequenzbereich: 504
- Frequenzbereich: 506
- Spektrum: 700
- Spektrum: 702
- Spektrum: 704

## Patentansprüche

1. Verfahren zur Bestimmung von Methylen-Diphenyl-Diisocyanat (MDI)-Isomerenkonzentrationen in einem Methylen-Diphenyl-Diisocyanat-Isomerengemisch, wobei
a) ein Nah-Infrarot(NIR)-Spektrum des Methylen-Diphenyl-Diisocyanat-Isomerengemisches aufgenommen wird,
b) das Spektrum in einem chemometrischen Kalibrationsmodell eingegeben wird, und
c) das Spektrum in dem chemometrischen Kalibrationsmodell in einem Frequenzbereich von 6250 - 6080 cm⁻¹ oder 5840 - 5650 cm⁻¹ ausgewertet wird.

2. Verfahren nach Anspruch 1 mit einem Methylen-Diphenyl-Diisocyanat-Isomerengemisch aus 2,4'MDI und 4,4'-MDI.

3. Verfahren nach einem der vorhergehenden Ansprüche 1 bis 2, **dadurch gekennzeichnet, dass** die Aufnahme des Spektrums ohne Probenahme online, insbesondere inline erfolgt.

4. Verfahren nach einem der vorhergehenden Ansprüche 1 bis 3, wobei das Ergebnis der Bestimmung der Isomerenzusammensetzung zur Steuerung und/oder Regelung verwendet wird, vorzugsweise zur automatischen Regelung.

5. Verfahren nach einem der vorhergehenden Ansprüche 1 bis 4, **dadurch gekennzeichnet, dass** die Isomerenkonzentrationen in dem Isomerengemisch im Bereich von 0,01 % bis 99,99 % liegen.

6. Verfahren nach einem der vorhergehenden Ansprüche 1 bis 5, **dadurch gekennzeichnet, dass** die Auswertung des Spektrums mit einem chemometrischen Modell mit einem Partial Least Square-Verfahren erfolgt.

7. Verfahren zur Regelung einer Isomerenanlage zur Herstellung eines Methylen-Diphenyl-Diisocyanat-Isomerengemisches mit einer Soll-Isomeren-Zusammensetzung, mit unter Anwendung des Verfahrens nach einem der vorhergehenden Ansprüche
- Aufnahme eines Nah-Infrarot(NIR)-Spektrum, des Methylen-Diphenyl-Diisocyanat-Isomerengemisches,
- Eingabe des Spektrums in ein chemometrisches Kalibrationsmodell zur Bestimmung einer Ist-Isomeren-Zusammensetzung des Isomerengemischs,
wobei die Auswertung der Spektrums des Methylen-Diphenyl-Diisocyanat-Isomerengemisches in dem chemometrischen Kalibrationsmodell in einem Frequenzbereich von 6250-6080cm⁻¹ oder 5840-5650cm⁻¹ erfolgt
- anschliessender Bestimmung einer Soll-Ist-Abweichung, und
- Regelung eines oder mehrerer Prozessparameter der Isomerenanlage basierend auf der Soll-Ist-Abweichung.

8. Verfahren nach Anspruch 7, wobei die Herstellung des Isomerengemischs in der Isocyanat-Isomerenanlage destillativ oder mittels Kristallisation erfolgt.

9. Verfahren nach einem der vorhergehenden Ansprüche 7 bis 8, **dadurch gekennzeichnet, dass** die Messung des Spektrums online, insbesondere inline erfolgt.

## Claims

1. A method of determining methylene diphenyl diisocyanate (MDI) isomer concentrations in a methylene diphenyl diisocyanate isomer mixture, wherein
a) a near infrared (NIR) spectrum of the methylene diphenyl diisocyanate isomer mixture is recorded,
b) the spectrum is entered into a chemometric calibration model, and
c) the spectrum is evaluated in the chemometric calibration model in a frequency range of 6250 - 6080 cm⁻¹ or 5840 - 5650 cm⁻¹.

2. The method according to claim 1, with a methylene diphenyl diisocyanate isomer mixture of 2,4'-MDI and 4,4'-MDI.

3. The method according to one of the preceding claims 1 to 2, **characterised in that** the recording of the spectrum takes place online, particularly inline, without sampling.

4. The method according to one of the preceding claims 1 to 3, wherein the result of the determination of the isomer composition is used for control and/or regulation, preferably for automatic regulation.

5. The method according to one of the preceding claims 1 to 4, **characterised in that** the isomer concentrations in the isomer mixture are in the range of from 0.01% to 99.99%.

6. The method according to one of the preceding claims 1 to 5, **characterised in that** the evaluation of the spectrum takes place with a chemometric model using a partial least square method.

7. A method of regulating an isomer plant for the production of a methylene diphenyl diisocyanate isomer mixture with a target isomer composition using the method according to one of the preceding claims:
- recording a near infrared (NIR) spectrum of the methylene diphenyl diisocyanate isomer mixture,
- entering the spectrum into a chemometric calibration model to determine an actual isomer composition of the isomer mixture, the evaluation of the spectrum of the methylene diphenyl diisocyanate isomer mixture in the chemometric calibration model taking place in a frequency range of 6250 - 6080 cm⁻¹ or 5840 - 5650 cm⁻¹,
- subsequent determination of a target/actual deviation and
- regulation of one or more process parameters of the isomer plant based on the target/actual deviation.

8. The method according to claim 7, wherein the production of the isomer mixture in the isocyanate isomer plant takes place by distillation or crystallisation.

9. The method according to one of the preceding claims 7 to 8, **characterised in that** the measurement of the spectrum takes place online, particularly inline.

## Revendications

1. Procédé pour déterminer les concentrations des isomères de méthylènediphényldiisocyanate (MDI) dans un mélange d'isomères de méthylènediphényldiisocyanate, où
a) on relève un spectre infrarouge proche (NIR) du mélange d'isomères de méthylènediphényldiisocyanate,
b) on introduit le spectre dans un modèle d'étalonnage chimio-métrique, et
c) on évalue le spectre dans le modèle d'étalonnage chimio-métrique dans une gamme de fréquences de 6250-6080 cm⁻¹ ou de 5840-5650 cm⁻¹.

2. Procédé selon la revendication 1, pour un mélange d'isomères de méthylènediphényldiisocyanate constitué de 2,4'-MDI et de 4,4'-MDI.

3. Procédé selon l'une des revendications précédentes 1 à 2, **caractérisé en ce que** le relevé du spectre est réalisé sans prélèvement d'échantillon, online, en particulier inline.

4. Procédé selon l'une des revendications précédentes 1 à 3, où le résultat de la détermination de la composition en isomères est utilisé pour la commande et/ou le réglage, de préférence pour un réglage automatique.

5. Procédé selon l'une des revendications précédentes 1 à 4, **caractérisé en ce que** les concentrations en isomère dans le mélange d'isomères se situent dans l'intervalle allant de 0,01% à 99,99%.

6. Procédé selon l'une des revendications précédentes 1 à 5, **caractérisé en ce que** l'évaluation du spectre est réalisée avec un modèle chimio-métrique avec un procédé Partial Least Square.

7. Procédé de réglage d'une installation pour la préparation d'un mélange d'isomères de méthylènediphényldiisocyanate avec une composition prescrite en isomères, en utilisant le procédé selon l'une des revendications précédentes, comprenant
- le relevé d'un spectre infrarouge proche du mélange d'isomères de méthylènediphényldiisocyanate,
- l'introduction du spectre dans un modèle d'étallonage chimio-métrique pour déterminer la composition réelle du mélange d'isomères,
où l'évaluation du spectre du mélange d'isomères de méthylènediphényldiisocyanate est réalisée dans le modèle d'étalonnage chimio-métrique dans une gamme de fréquences de 6250-6080 cm⁻¹ ou de 5840-5650 cm⁻¹,
- puis la détermination de l'écart entre les chiffres prescrits et les chiffres réels et
- le réglage d'un ou de plusieurs paramètres de fonctionnement de l'installation sur base de l'écart entre les chiffres prescrits et les chiffres réels.

8. Procédé selon la revendication 7, où la préparation du mélange d'isomère est réalisée dans l'installation d'isocyanate, par distillation ou cristallisation.

9. Procédé selon l'une des revendications précédentes 7 à 8, **caractérisé en ce que** la mesure du spectre est réalisée online, en particulier inline.
